(19) Europäisches Patentamt
European Patent Office
Office européen des brevets

(11) Veröffentlichungsnummer: **0 011 098**
**A1**

# (12) EUROPÄISCHE PATENTANMELDUNG

(21) Anmeldenummer: **79103323.6**

(22) Anmeldetag: **07.09.79**

(51) Int. Cl.³: **C 07 C 45/32**
C 07 C 49/00

(30) Priorität: **08.11.78 DE 2848400**

(43) Veröffentlichungstag der Anmeldung:
**28.05.80 Patentblatt 80/11**

(84) Benannte Vertragsstaaten:
**BE FR GB IT NL**

(71) Anmelder: **CHEMISCHE WERKE HÜLS AG**
**- RSP Patente PB 15 - Postfach 13 20**
**D-4370 Marl 1(DE)**

(72) Erfinder: **Scharf, Helmut, Dr.**
**Schetterstrasse 82**
**D-4235 Schermbeck(DE)**

## (54) Verfahren zur Herstellung von Ketonen aus Aldehyden.

(57) In einem Verfahren zur Herstellung von Ketonen mit mindestens 4 C-Atomen und mit der allgemeinen Formel

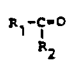

in der $R_1$ und $R_2$ lineare oder verzweigte Alkyl-, Alkenyl-, Alkylaryl-, Alkenylaryl-, Alkylcycloalkyl-, Alkenyl- cycloalkyl-, Alkylcycloalkenyl- oder Alkenylcycloalkenyl- reste mit 1 bis 18 C-Atomen oder zusammen einen makrocyclischen Ring mit 5 bis 18 C-Atomen darstellen und die Reste Gruppen mit Heteroatomen enthalten können, setzt man Aldehyde mit der allgemeinen Formel

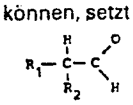

in der $R_1$ und $R_2$ die oben angegebenen Reste darstellen, in der Gasphase in einer Konzentration des Alkehyds im Eingangsgas von 1 bis 8 Volumenprozent mit Sauerstoff bzw. sauerstoffhaltigen Gasen bei Temperaturen von 120 bis 270°C und Kontaktzeiten von 0,2 bis 10 Sekunden, gegebenenfalls in Gegenwart von inerten Verdünnungsmitteln, an Metalloxidkatalysatoren um, die ein Oxid der Elemente Kupfer und oder Mangan und gegebenenfalls Zinkoxid und oder Graphit enthalten. Insbesondere läßt sich so Heptanon-3 aus 2-Ethylhexanal herstellen.

EP 0 011 098 A1



BAD ORIGINAL

Verfahren zur Herstellung von Ketonen

Die selektive Herstellung eines Ketons durch heterogen katalysierte, oxidative Decarbonylierung eines um ein Kohlenstoffatom reicheren Aldehyds in der Gasphase ist für Aceton bekannt (DE-Patentanmeldung 28 02 672.4). Bei der Herstellung von Aceton wird Isobutanal in der Gasphase an Metalloxidkatalysatoren oxidativ zu Aceton decarbonyliert. In der Literatur sind keine weiteren Verfahren bekannt, bei denen Aldehyde in der Gasphase selektiv zu den entsprechenden um ein Kohlenstoffatom ärmeren Ketonen oxidativ decarbonyliert werden, so daß man annehmen mußte, daß die hohe Selektivität bei der oxidativen Decarbonylierung eines Aldehyds in der Gasphase auf den einen Fall Isobutanal/Aceton beschränkt ist. Diese Annahme wurde sogar durch eine Reihe eigener Versuche mit anderen Aldehyden zunächst bestätigt (Vergleichsbeispiele 5 bis 9).

Daraus ergab sich die Aufgabe, ein Verfahren zur selektiven Herstellung von Ketonen mit mindestens 4 C-Atomen zu finden.

Diese Aufgabe wird erfindungsgemäß gelöst, indem man zur Herstellung von Ketonen mit mindestens 4 C-Atomen und mit der allgemeinen Formel 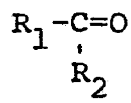 ,

in der $R_1$ und $R_2$ lineare oder verzweigte Alkyl-, Alkenyl-, Alkylaryl-, Alkenylaryl-, Alkylcycloalkyl-, Alkenylcycloalkyl-, Alkylcycloalkenyl- oder Alkenylcycloalkenylreste mit 1 bis 18 C-Atomen oder zusammen einen makrocyclischen Ring mit 5 bis 18 C-Atomen darstellen und die Reste Gruppen mit Heteroatomen enthalten können, Aldehyde mit der allgemeinen Formel

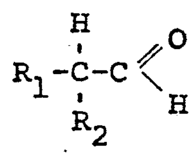

in der $R_1$ und $R_2$ die oben angegebenen Reste darstellen, in der Gasphase in einer Konzentration des Aldehyds im Eingangsgas von 1 bis 8 Volumenprozent mit Sauerstoff bzw. sauerstoffhaltigen Gasen bei Temperaturen von 120 bis 270 °C und Kontaktzeiten von 0,2 bis 10 Sekunden, gegebenenfalls in Gegenwart von inerten Verdünnungsmitteln, an Metalloxidkatalysatoren umsetzt, die ein Oxid der Elemente Kupfer und/oder Mangan und gegebenenfalls Zinkoxid und/oder Graphit enthalten.

Überraschenderweise wurde gefunden, daß die selektive oxidative Decarbonylierung von Aldehyden zu Ketonen in der Gasphase nicht auf Isobutanal/Aceton beschränkt ist, sondern ein allgemeines Reaktionsprinzip darstellt, wenn als Katalysatoren Kupfer- und/oder Manganoxid und solche Aldehyde verwendet werden, die in $\alpha$-Stellung zur Carbonylgruppe ein tertiäres Kohlenstoffatom mit einem Wasserstoffatom besitzen:

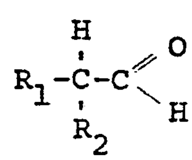

Die Reste $R_1$ und $R_2$ können Alkyl-, Alkenyl-, Alkylaryl-, Alkenylaryl-, Alkylcycloalkyl-, Alkenylcycloalkyl-, Alkylcycloalkenyl- oder Alkenylcycloalkenylreste mit 1 bis 18 C-Atomen darstellen. Die Reste $R_1$ und $R_2$ können zusammen auch einen makrocyclischen Ring mit 5 bis 18 C-Atomen bilden. Die Begrenzung der Reste bezüglich ihrer C-Zahl ist nicht bedingt durch den Reaktionsmechanismus, den Umsatz oder die Selektivität, sondern durch die Notwendigkeit, die Aldehyde zu verdampfen. Bei entsprechend niedrigen Drücken können also auch Aldehyde mit mehr C-Atomen eingesetzt werden. Da in der Praxis Aldehyde mit Resten, die mehr als 18 C-Atome enthalten, seltener vorkommen und diese Aldehyde schwer in die Gasphase zu überführen sind, werden Aldehyde mit Resten, die nicht mehr als 18 C-Atome enthalten, bevorzugt.

Die Reste $R_1$ und $R_2$ können auch Heteroatome, wie z. B. Si, N, P, As, O, S, Se, F, Cl, Br oder J etc., enthalten, wobei die Heteroatome je nach ihrer Art für sich allein an einem oder mehreren C-Atomen, wie z. B. -C-Cl, 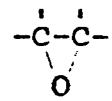 etc., zwischen zwei oder mehreren C-Atomen, wie z. B. -C-N=C-, 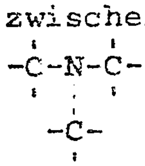 etc., oder in Verbindung mit weiteren Atomen als Gruppe an einem oder mehreren C-Atomen wie z. B. $-C-COOCH_3$, 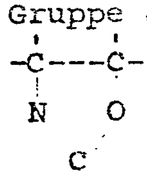 etc., oder zwischen zwei oder mehreren C-Atomen, wie z. B. 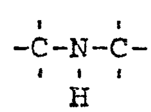 etc., sitzen können.

Die Heteroatomgruppen können je nach ihrer Art während der oxidativen Decarbonylierung inert bleiben oder aber selbst Reaktionen eingehen. Wegen der Vielzahl an möglichen Gruppen mit Heteroatomen in den Resten $R_1$ und $R_2$ muß im Einzelfall getestet werden, ob die Gruppe mit den Hetero-

BAD ORIGINAL

atomen die Selektivität bei der oxidativen Decarbonylierung des Aldehyds beeinträchtigt. Das Verfahren eignet sich insbesondere zur oxidativen Decarbonylierung von 2-Ethyl-hexanal zu Heptanon-3.

Die erfindungsgemäßen Katalysatoren lassen sich als Voll- oder Trägerkatalysatoren nach den üblichen Methoden herstellen. Bevorzugt werden Trägerkatalysatoren, da sie mechanisch stabiler und billiger sind. Der Kupfer- und/oder Mangangehalt des fertigen Katalysators soll bevorzugt 0,1 bis 6 Gewichtsprozent betragen. Unter 0,1 Gewichtsprozent wird der Umsatz für technische Zwecke zu niedrig und Konzentrationen über 6 Gewichtsprozent bringen keine weitere Verbesserung mehr. Außerdem nimmt bei höheren Konzentrationen der Anteil an Totalverbrennung des Aldehyds zu. Besonders bevorzugt wird ein Gehalt an Kupfer und/oder Mangan in Form der Oxide von 1 bis 3 Gewichtsprozent. Der Zinkgehalt des fertigen Katalysators in Form des Oxids kann 0,1 bis 80 Gewichtsprozent betragen. In der Regel werden Gehalte von 0,1 bis 20 Gewichtsprozent verwendet. Besonders bevorzugt werden Zinkgehalte von 1 bis 5 Gewichtsprozent. Bei Zinkgehalten größer 20 Gewichtsprozent dient das Zinkoxid in der Regel gleichzeitig als Träger für die anderen Katalysatorkomponenten. In diesem Fall hat es sich als vorteilhaft erwiesen, dem Zinkoxid 2 bis 20 Gewichtsprozent Kolloidgraphit, bezogen auf das Zinkoxid, hinzuzufügen. Bevorzugt werden Graphitgehalte von 2 bis 5 Gewichtsprozent, bezogen auf das Zinkoxid, da größere Mengen an Graphit keine Vorteile mehr bringen.

Als Träger für die aktiven Metalloxide kommen Materialien in Frage, die unter den Reaktionsbedingungen entweder inert oder aber eine Eigenaktivität für die oxidative Decarbonylierung aufweisen, wie dies z. B. bei Zinkoxid-Graphit der Fall ist. Als besonders geeignete Trägermaterialien haben sich handelsübliches Aluminium-, Zink- und Titandioxid erwiesen. Wegen der Vielfalt der Unterschiede bei den einzelnen Trägermaterialien, z. B. bei den verschiedenen Aluminiumoxiden, ist es notwendig, den speziellen Träger unter Reaktionsbedingungen auf eine selektivitätsmindernde Eigenaktivität zu prüfen.

Das Aufbringen der katalytischen Komponenten auf den Träger erfolgt nach den in der Technik üblichen Methoden, wie z. B. Imprägnieren der Träger mit wäßrigen Salzlösungen der Metalle, Trocknen der imprägnierten Träger und Umwandlung der Salze bei erhöhten Temperaturen zu den entsprechenden Oxiden. Hierbei kann das Aufbringen der Metallverbindungen auf den Träger in einem Schritt erfolgen, indem Lösungen verwendet werden, die alle erforderlichen Metallionen enthalten, oder aber in einzelnen Schritten, indem zunächst die eine Metallverbindung auf den Träger gebracht wird, das Produkt getrocknet und kalziniert wird und anschließend die nächste Komponente mit den gleichen Verarbeitungsschritten etc. Als äußere Form des Katalysators sind die Kugel-, Tabletten-, Strang-, Pillen- oder Stückform geeignet.

Die Konzentration des Aldehyds in dem Gasgemisch kann zwischen 1 und 8 Volumenprozent schwanken. Es sind aber auch höhere und niedrigere Konzentrationen möglich, jedoch sind die kleinen Konzentrationen wirtschaftlich uninteressant, und bei den höheren Konzentrationen wird das Problem der Wärmeabfuhr schwierig. Besonders bevorzugt werden Konzentrationen zwischen 2 und 5 Volumenprozent.

Die Sauerstoffkonzentration in dem Eingangsgas richtet sich nach der Aldehydkonzentration. Das kleinste molare Verhältnis sollte 1 : 1,2 betragen. Bei noch kleineren Verhältnissen nimmt der Umsatz rasch ab. Bevorzugt werden molare Verhältnisse von Aldehyd : Sauerstoff von 1 : 2 bis 1 : 3. Das Aldehyd-Sauerstoff-Verhältnis kann aber auch über diese Verhältnisse hinausgehen. So läßt sich die Reaktion

auch mit reinem Sauerstoff durchführen, wenn die Aldehydkonzentration nicht zu hoch gewählt wird. Bevorzugt setzt man als Oxidationsmittel Luft ein. In der Regel wird dem Eingangsgas ein inertes Verdünnungsmittel zugegeben. Als Verdünnungsmittel kommen in Frage: Stickstoff, Wasserdampf, Kohlendioxid oder Kohlenmonoxid. Bevorzugte Verdünnungsmittel sind Stickstoff und/oder Wasserdampf, wobei der Stickstoff aus der als bevorzugtes Oxidationsmittel verwendeten Luft stammt.

Die Reaktionstemperatur richtet sich nach dem verwendeten Aldehyd. Sie beträgt 120 bis 270 °C. Die Verweilzeit der Eingangsstoffe in dem Katalysatorbett (Kontaktzeit) richtet sich nach der Zusammensetzung des Eingangsgemisches und der Reaktionstemperatur und kann innerhalb eines Bereiches von 0,2 bis 10 sec schwanken. Sie liegt vorzugsweise in einem Bereich von 1 bis 6 sec. Die Umsetzung wird normalerweise bei Normaldruck oder leicht erhöhten Drücken bis zu ca. 5 bar durchgeführt. Es sind aber auch höhere Drücke anwendbar. Bei höher siedenden Aldehyden wird die Reaktion im Vakuum durchgeführt. Die Höhe des Vakuums richtet sich nach dem verwendeten Aldehyd.

Mit Hilfe des erfindungsgemäßen Verfahrens ist es möglich, bisher schwer zugängliche Ketone auf einfache Weise selektiv herzustellen.

Beispiel 1 (Katalysatorherstellung)
200 g aktiviertes Aluminiumoxid (Stränge, Ø 1,6 mm, Länge 4 mm, Katalysatorenwerke Houdry-Hüls) imprägniert man mit 213 g einer wäßrigen Kupfertetramincarbonatlösung, die 4,3 Gewichtsprozent Kupferionen enthält. Die überstehende Lösung läßt man ab. Der Katalysator wird 16 h bei 110 °C getrocknet und 4 h bei 350 °C kalziniert. Der Kupfergehalt in Form des Oxids beträgt 2,5 Gewichtsprozent, bezogen auf den fertigen Katalysator.

Beispiel 2 (Katalysatorherstellung)
112 g aktiviertes Aluminiumoxid werden mit 98 $cm^3$ einer wäßrigen Lösung, die 20,5 g $Zn(NO_3)_2 \cdot 6 H_2O$ enthält, imprägniert, 16 h bei 110 °C getrocknet und 16 h bei 350 °C kalziniert. Der kalzinierte Katalysator wird anschließend mit 98 $cm^3$ einer wäßrigen Kupfertetramincarbonatlösung, die 14,1prozentig an Kupferionen ist, imprägniert und wieder 16 h lang bei 110 °C getrocknet und 16 h bei 350 °C kalziniert. Der fertige Katalysator enthält 2,3 Gewichtsprozent Kupfer und 2,3 Gewichtsprozent Zink in Form ihrer Oxide.

Beispiel 3 (Katalysatorherstellung)
Man mischt 960 g Zinkoxid innig mit 40 g Kolloidgraphit und 30 g Wasser und preßt zu Tabletten (Durchmesser 4 mm, Dicke 4 mm). Anschließend trocknet man 16 h bei 110 °C und tempert 16 h bei 350 °C. Die fertigen Tabletten imprägniert man mit 122 g Kupfertetramincarbonatlösung, die 13,9 Gewichtsprozent Kupferionen enthält, trocknet 16 h bei 110 °C und kalziniert 16 h bei 350 °C. Der Kupferoxidgehalt des fertigen Katalysators beträgt 2,1 Gewichtsprozent, der Graphitgehalt 3,9 Gewichtsprozent.

Beispiel 4 (Katalysatorherstellung)
85 g käufliches Zinkoxid und 10 g käuflicher Kolloidgraphit suspendiert man in 120 $cm^3$ einer wäßrigen Manganacetatlösung, die 5 g Manganionen enthält. Die Suspension wird bis zur Trockne eingedampft und der Rückstand wird 16 h bei 110 °C getrocknet, gemahlen, zu Tabletten gepreßt (Ø 4 mm, Höhe 4 mm) und 16 h bei 350 °C getempert. Der Mangangehalt in Form des Oxids beträgt 5 Gewichtsprozent, bezogen auf den fertigen Katalysator.

Beispiele 5 bis 9 (Vergleichsbeispiele)
60 $cm^3$ des in Beispiel 2 beschriebenen Katalysators gibt man in ein mit siedendem Wasser thermostatisiertes Reaktionsrohr aus Stahl mit einem inneren Durchmesser von 20 mm und beaufschlagt bei den in Tabelle 1 angegebenen Temperaturen und Kontaktzeiten von 2 sec bei Reaktorinnendrücken von 1,5 bar mit Gasgemischen, die aus 2,5 Volumenprozent eines Aldehyds, der in α-Stellung zur Carbonylgruppe kein tertiäres C-Atom mit einem Wasserstoffatom besitzt, 44,5 Volumenprozent Luft und 52,0 Volumenprozent Wasserdampf bestehen. Die den Reaktor verlassenden Gasgemische untersucht man gaschromatographisch. Die erhaltenen Ergebnisse sind in der Tabelle 1 zusammengefaßt.

<u>Beispiele 10 bis 14</u>

Unter den in den Beispielen 5 bis 9 genannten Versuchsbedingungen setzt man Aldehyde ein, die in α-Stellung zur Carbonylgruppe ein tertiäres C-Atom mit einem Wasserstoffatom besitzen. Die erhaltenen Ergebnisse sind in der Tabelle 2 aufgeführt.

Tabelle 1 (Vergleichsbeispiele)

| Beispiel | Aldehyd | Reaktionstemperatur °C | Keton | Bemerkungen |
|---|---|---|---|---|
| 5 | 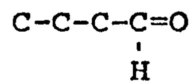 | 140<br>161 | -<br>- | keine Reaktion<br>Totalverbrennung |
| 6 | 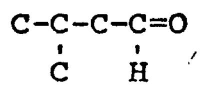 | 140 - 336 | - | Hauptprodukte<br>Aceton und Essigsäure |
| 7 | 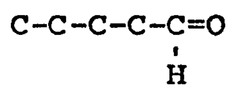 | 140<br>175 | -<br>- | Hauptprodukt Isopentanal<br>Totalverbrennung |
| 8 | 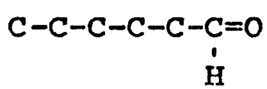 | 172 | - | Hauptprodukt Capronsäure |
| 9 | 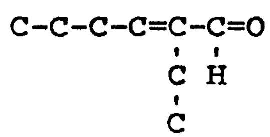 | 170 | - | keine Reaktion |

Tabelle 2

| Beispiel | Aldehyd | Reaktionstemperatur °C | Keton | Umsatz (%) | Selektivität (Molprozent) |
|---|---|---|---|---|---|
| 10 | 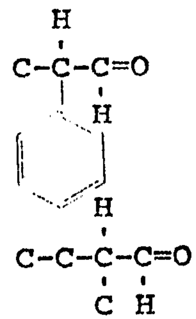 | 194 (Druck: 10 mbar) | 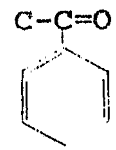 | 100 | 79 |
| 11 | | 216 | 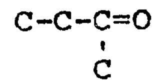 | 96 | 90 |
| 12 | 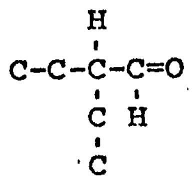 | 191 | 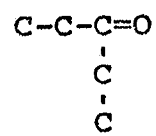 | 83 | 79 |
| 13 | 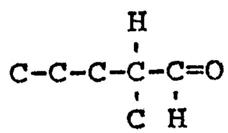 | 209 | 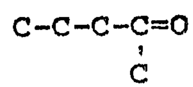 | 97 | 84 |
| 14 | 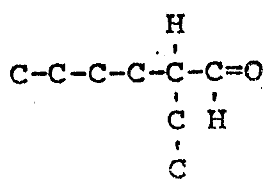 | 171 | 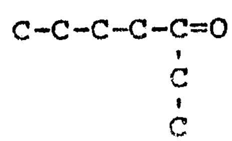 | 97 | 87 |

0011098

Patentansprüche:

1. Verfahren zur Herstellung von Ketonen mit mindestens 4 C-Atomen und mit der allgemeinen Formel

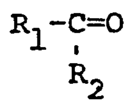

in der $R_1$ und $R_2$ lineare oder verzweigte Alkyl-, Alkenyl-, Alkylaryl-, Alkenylaryl-, Alkylcycloalkyl-, Alkenylcycloalkyl-, Alkylcycloalkenyl- oder Alkenylcycloalkenylreste mit 1 bis 18 C-Atomen oder zusammen einen makrocyclischen Ring mit 5 bis 18 C-Atomen darstellen und die Reste Gruppen mit Heteroatomen enthalten können,
dadurch gekennzeichnet,
daß Aldehyde mit der allgemeinen Formel

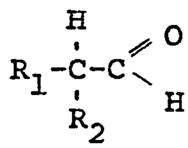

in der $R_1$ und $R_2$ die oben angegebenen Reste darstellen, in der Gasphase in einer Konzentration des Aldehyds im Eingangsgas von 1 bis 8 Volumenprozent mit Sauerstoff bzw. sauerstoffhaltigen Gasen bei Temperaturen von 120 bis 270 $^{o}C$ und Kontaktzeiten von 0,2 bis 10 Sekunden, gegebenenfalls in Gegenwart von inerten Verdünnungsmitteln, an Metalloxidkatalysatoren umgesetzt werden, die ein Oxid der Elemente Kupfer und/oder Mangan und gegebenenfalls Zinkoxid und/oder Graphit enthalten.

2. Verfahren nach Anspruch 1,
dadurch gekennzeichnet,
daß die Menge an Kupfer und/oder Mangan in Form der Oxide 0,1 bis 6 Gewichtsprozent, bezogen auf den fertigen Katalysator, beträgt.

0011098

Europäisches Patentamt

# EUROPÄISCHER RECHERCHENBERICHT

Nummer der Anmeldung

EP 79103323.6

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | GB - A - 1 133 882 (IMPERIAL CHEMICAL INDUSTRIES)<br>+ Gesamt +<br>-- | 1,2 | C 07 C 45/32<br>C 07 C 49/00 |
| | AT - B - 339 874 (HÜLS)<br>+ Seite 2, 3.Absatz; Beispiele; Ansprüche +<br>-- | 1,2 | |
| | DE - B - 1 956 018 (MELLE-BEZONS)<br>+ Ansprüche 1 und 3; Beispiel 13 +<br>-- | 1,2 | RECHERCHIERTE SACHGEBIETE (Int. Cl. 3) |
| | DE - A - 2 157 307 (DAICLE LTD.)<br>+ Seite 2, 3. Absatz; Beispiel 1, Ansprüche 1 bis 9 +<br>-- | 1,2 | C 07 C 45/00<br>C 07 C 49/00 |
| P | DE - A1 - 2 738 269 (HÜLS)<br>+ Ansprüche 1 bis 4; Beispiel 1 +<br>& BE-A1- 869 916 & FR-A1-2 405915<br>& GB-A -2 003 044 & NL-A-7 808 746<br>---- | 1,2 | |

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 11-01-1980 | KÖRBER |